(19) Europäisches Patentamt

European Patent Office

Office européen des brevets

(11) EP 1 128 787 B1

(12) **EUROPEAN PATENT SPECIFICATION**

(45) Date of publication and mention
of the grant of the patent:
**11.01.2006 Bulletin 2006/02**

(21) Application number: **99957524.4**

(22) Date of filing: **12.11.1999**

(51) Int Cl.:
***A61F 2/28*** (2006.01)          ***A61N 7/00*** (2006.01)

(86) International application number:
**PCT/US1999/026265**

(87) International publication number:
**WO 2000/028925 (25.05.2000 Gazette 2000/21)**

(54) **PROSTHESIS FOR INDUCING BONY INGROWTH USING ULTRASOUND THERAPY**

PROTHESE ZUR INDUKTION DES KNOCHENWACHSTUMS DURCH ULTRASCHALLTHERAPIE

PROTHESE VISANT A INDUIRE UNE INTERPOSITION DU TISSU OSSEUX PAR THERAPIE ULTRASONORE

(84) Designated Contracting States:
**AT BE CH CY DE DK ES FI FR GB GR IE IT LI LU MC NL PT SE**

(30) Priority: **13.11.1998 US 108235 P**

(43) Date of publication of application:
**05.09.2001 Bulletin 2001/36**

(73) Proprietor: **Exogen, Inc.**
**Memphis, Tennessee 38116 (US)**

(72) Inventors:
• **TALISH, Roger, J.**
**Hillsborough, NJ 08876 (US)**

• **WINDER, Alan, A.**
**Westport, CT 06880 (US)**

(74) Representative: **Draggett, Peter Thornton et al**
**Group Patents & Trade Marks Dept.,**
**Smith & Nephew Group Research Centre,**
**York Science Park**
**Heslington,**
**York Y010 5DF (GB)**

(56) References cited:
**WO-A-90/06720          WO-A-98/34578**
**DE-A- 3 639 263          GB-A- 2 156 983**
**GB-A- 2 277 448          US-A- 5 330 481**
**US-A- 5 730 705**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

*Technical Field*

The present disclosure relates to prosthetic implant devices.

*Background*

Joint replacement, or arthroplasty, is a surgical procedure in which the diseased portions of the joint are removed and replaced with new artificial parts called a prosthesis. During typical joint replacement surgery, the surgeon removes the diseased portion of the bone, surrounding tissue and cartilage from the joint leaving the healthy parts of the joint intact. The surgeon then replaces the diseased portion of the joint with new parts which mimic the movement of the joint. For example, with hip arthroplasty, the surgeon replaces the head of the femur and the acetabulum with artificial parts made of materials which permit a natural, gliding motion of the hip joint. This generic type of device includes prostheses that have femoral components made of alloys, such as cobalt-chromium-molybdenum and/or titanium-based alloys.

In some cases the surgeon uses a special glue or cement to bond the new parts of the joint to the existing healthy bone. In other cases the artificial parts are made from a porous material which permits the patient's own bone to grow into the pores of the porous material to hold the new parts in place. See, e.g., U.S. Patent Nos. 4,536,894 to Galante et al., 5,018,285 to Zolman et al. and 5,004,476 to Cook.

Successful replacement of deteriorated, arthritic, and severely injured joints has contributed to enhanced mobility and comfortable, independent living for many people who would otherwise be substantially disabled. New technologies involving prosthetic devices for replacement of joints, along with advances in surgical techniques, has diminished the risks associated with these operations and improved the immediate and long-term outcome of joint replacement surgery.

Questions remain, however, concerning which prosthetic designs and materials are most effective for specific groups of patients and which surgical techniques and rehabilitation approaches yield the best long-term results. For example, patients whose joints have been severely damaged due to osteoporosis tend to suffer from long-term difficulties with the prosthesis due to insufficient bone mass surrounding the medullary canal. Osteoporosis causes abnormally porous and fragile bones due to age, low calcium intake, inadequate physical activity, certain drugs, estrogen deficiency, hormone disorders, nutritional disorders, bone disuse and family history of the disease. Implanting a prosthetic device into such porous and fragile bones has had limited success and may require a repeat/revision procedure or long therapy. Issues also exist regarding the best indications and approaches for revision surgery.

Nevertheless, further improvements in the total design of joint prostheses are needed to facilitate more stable fixation of the implanted prosthesis at the bone/metal interface. For example, with cemented prosthetic devices fixation problems can occur due to the various stress loads, i.e., the compression, shear and torsion to which the implanted device is subjected. These mechanical forces, especially shear and torsion, as well as other factors such as osteoporosis, weaken the bone-cement bond. In addition, it is known that there is a tendency for bone resorption which also weakens the cement bond between the intramedullary canal of the bone and the prosthesis.

By providing a bony ingrowth surface on the prosthetic device and/or by providing therapy for inducing bony ingrowth into the prosthetic device, a more stable fixation can be made. However, with conventional prosthetic device treatments/ techniques which include bony ingrowth surfaces, sufficient bony ingrowth for long term stabilization typically requires the prosthesis to be stably fixed for at least six weeks after surgery, and any relative motion of the prosthesis during that period prevents or minimizes bony ingrowth. This is a particularly significant problem in view of the difficulty in fitting the prosthesis with sufficiently dose tolerances to provide large contact areas between the porous material and the bone, even where the entire outer surface of the prosthesis is fabricated from porous material. For example, it has been reported that an instance of 10 to 20 percent of femoral stem loosening or failure in total hip arthroplasty patients followed over five or more years, especially in younger patients.

US 5330481 discloses a prosthesis with a Morse taper and a vertical tapped hole. US-A-5330481 discloses a prosthesis comprising the features as defined in the preamble of claim 1.

The present disclosure also relates to directing ultrasonic energy in relatively low levels into living tissue to stimulate ingrowth of the soft bone surrounding the medullary canal into the prosthesis. The disclosure also includes various techniques for the transdermal delivery of acoustic energy through body tissue and/or fluids to propagate resonant waves along the inner cavity and/or outer surface of the prosthesis to stimulate ingrowth of the surrounding soft cancellous bone about the periphery of the medullary canal.

Acoustic bone fracture repair techniques and parameter preferences are discussed in detail in U.S. Patent No. 5,520,612. Much like bone fracture repair, acoustic techniques to stimulate bony ingrowth are also subject to a range of values best determined by professional experience. However, it is nevertheless helpful to list certain parameter considerations which may play a significant role in promoting ingrowth:

1) The frequency of surgically non-invasive acoustic delivery into the body should be carefully calculated and monitored to insure steady standing-wave development within the medullary canal;

2) The frequency of the transducer (or other carrier) should be adjustably selectable, with provision for frequency sweeping between adjusted limits; and

3) The frequency of pulse modulation should be adjustably selectable, with provision for frequency sweeping between adjusted limits.

## SUMMARY

The invention is as claimed in the claims.

A bone prosthesis indudes a first portion for engaging a first bone segment and at least one channel disposed within the first portion for propagating acoustic energy through the channel to the first bone segment. Preferably, the prosthesis further includes a second portion for engaging a second bone segment. The channel includes an interior reflective surface which defines a resonating chamber disposed through the first portion. In one embodiment, the resonating chamber includes at least one opening for receiving acoustic energy. In another embodiment, the resonating chamber is convoluted.

Another embodiment of the bone prosthesis includes a first portion for engaging a first bone segment and a second portion for engaging a second bore segment. At least one of the portions includes at least one means for propagating acoustic energy to the corresponding bone segment. Preferably, the propagating means includes; a transducer collar which engages one of the portions; a transducer disposed adjacent one of the portions; and/or a piezoelectric/piezoceramic membrane material which is disposed between a porous material wrapped around the prosthesis and the outer periphery of the prosthesis.

In another embodiment, the bone prosthesis includes a ball portion for engaging the acetabulum of the pelvic bone and the first portion is an implant for engaging the medullary canal of the femur. In yet another embodiment, the first portion engages the medullary canal of the humerus and a second portion engages the medullary canal of the ulna and the first and second portions move relative to one another about a pivot.

In still another embodiment, the first portion engages the femur and the second portion engages the tibia. The first and second portions are movable relative to one another upon movement of one of the femur and the tibia. Preferably, the first portion includes at least one dowel which engages a corresponding bore associated with the femur and the second portion includes at least one dowel which engages a corresponding bore associated with the tibia. The channel includes an interior reflective surface which defines a resonating chamber disposed through each of the dowels.

## BRIEF DESCRIPTION OF THE DRAWINGS

Fig. 1A is a front view of one embodiment of the present disclosure showing a hip prosthesis implanted within the upper femur with an external transducer emitting acoustic waves at the prosthesis to stimulate bony ingrowth;

Fig. 1B is partial cross-section of the hip prosthesis of Fig. 1 showing an internal reflective surface and a resonating chamber;

FIG. 1C is partial cross-section of the hip prosthesis of Fig. 1 showing the external transducer emitting acoustic waves at the reflective surface which, in turn, directs the waves downward through the resonating chamber;

Fig. 2A is partial cross-section of an alternate embodiment of the hip prosthesis of Fig. 1 showing an internally disposed transducer mounted within the resonating chamber;

Fig. 2B is partial cross-section of the hip prosthesis of Fig. 2A showing the external transducer emitting acoustic waves at the internal transducer which, in turn, emits acoustic waves downward through the resonating chamber;

Fig. 3 is partial cross-section of an alternate embodiment of the hip prosthesis of Fig. 1 showing a resonating chamber which is configured and dimensioned in a convoluted form to maximize the interference between the acoustic wave and the internal walls of the resonating chamber;

Fig. 4 is partial cross-section of an alternate embodiment of the hip prosthesis of Fig. 1 showing a series of laterally extending slots which extend from the interior walls of the resonating chamber to the outermost periphery of the prosthesis to conduct energy directly to the inner walls of the medullary canal;

Fig. 5A is partial cross-section of an alternate embodiment of the hip prosthesis of Fig. 1 showing a transducer collar which surrounds the prosthesis and emits acoustic waves downwardly along the outer periphery of the prosthesis to stimulate bony ingrowth from the surrounding bone of the medullary canal;

Fig. 5B is partial cross-section of an alternate embodiment of Fig. 5A showing a hip prosthesis having both an internally disposed resonating chamber for internally propagating acoustic waves and a transducer collar for emitting acoustic waves downwardly along the outer periphery of the prosthesis;

Fig. 6A is a front view of an alternate embodiment of the hip prosthesis of Fig. 1 showing a piezoelectric/piezoceramic membrane material disposed between a porous coating and the outer shell of the hip prosthesis for conducting acoustic energy to the medullary canal;

Fig. 6B is a cross section of the Fig. 6A embodiment taken along line 6B-6B;

Figs. 7A-7E are front views of various embodiments of the hip prosthesis of Fig. 1 showing various patterns disposed about the outer periphery of the prosthesis for promoting acoustic wave propagation to the medullary canal;

Fig. 8 is a front view of a diagnostic apparatus having a main transmitting unit and a send/receive probe for monitoring and recording the acoustic signals propagated through the hip prosthesis and medullary canal;

Fig. 9 is a front view of an alternate embodiment of the diagnostic apparatus having a main transmitting unit, a probe for sending acoustic waves through the hip prosthesis and a receiving sensor array probe for monitoring the acoustic signals propagated through the hip prosthesis and relaying the information back to the main unit;

Figs. 10A-10J show various views of an alternate embodiment of the present disclosure showing a knee joint prosthesis implanted between the lower femur and the upper portion of the tibia with an external transducer emitting acoustic waves at the prosthesis to stimulate bony ingrowth;

Figs. 11A and 11B show an alternate embodiment of a knee joint prosthesis having a plurality of dowels each having an inwardly disposed resonating chamber for propagating acoustic energy therethrough to stimulate bony ingrowth;

Figs. 12A-12C show various views of an alternate embodiment of the present disclosure showing an elbow joint prosthesis implanted between the humerus and the ulna with an external transducer emitting acoustic waves at the prosthesis to stimulate bony ingrowth; and

Figs. 13A-13C show an alternate embodiment of an elbow joint prosthesis having a plurality of inwardly disposed resonating chambers for propagating acoustic energy therethrough to stimulate bony ingrowth.

## DETAILED DESCRIPTION OF THE PREFERRED EMBODIMENTS

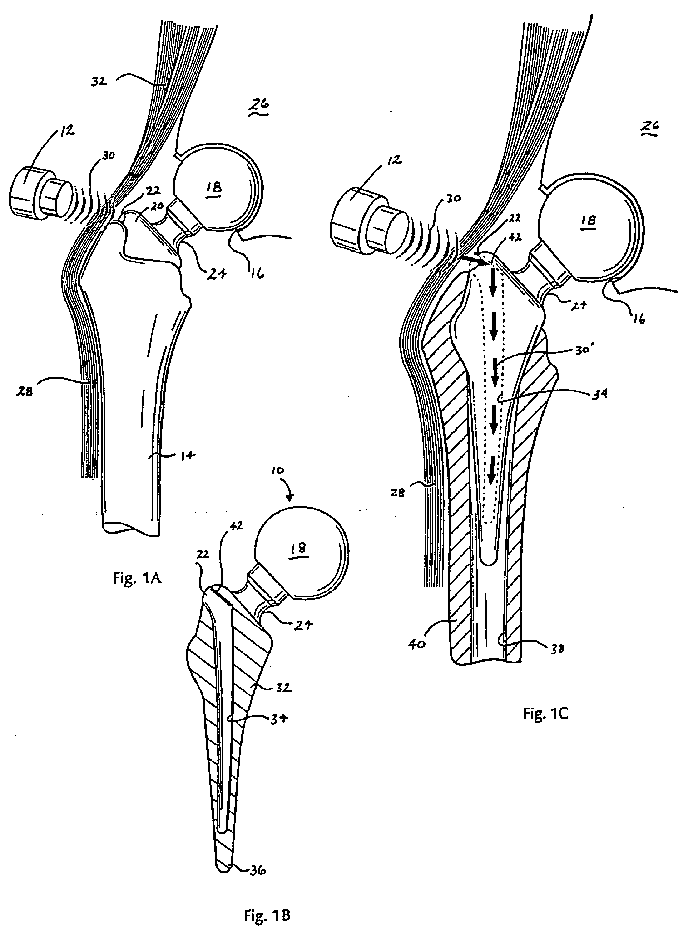

Referring now to Figs. 1A-1C which show one embodiment of the present disclosure, namely, a hip prosthesis which is generally designated by reference numeral 10. Hip prosthesis 10 includes a head or ball portion 18 which is connected to a lower wedge-like implant member 32 by way of a neck portion 24. Preferably, the lower wedge member 32 is generally tapered such that the lowermost portion 36 is configured to facilitate insertion of the wedge member 32 into the medullary canal 38 of the femur bone 14.

As best seen in Fig. 1A, the upper end "ball" portion 18 of the prosthesis is preferably configured and dimensioned to engage the acetabulum (socket) 16 of the pelvic bone 26 in a cup-like manner. This "ball and socket" arrangement allows a wide range of motion, including sitting, standing, walking and other daily activities. Once the "ball and socket" are engaged, the muscles and ligaments 28, 32 of the upper leg, e.g., vastus lateralis and gluteus muscles, among other things, cooperate to retain the hip joint in place in much the same fashion as the ball and socket-like arrangement of the original hip.

Figs. 1A and 1C show the preferred position of the wedge member 32 implanted within the femur 14. More particularly, during hip replacement surgery, the surgeon removes the diseased portion of the bone, surrounding tissue and cartilage from the hip joint leaving the healthy parts of the hip joint intact. The upper portion of the femur bone 14 is preferably surgically reconfigured to expose the medullary canal 38 which is a generally centrally located passageway disposed within the femur 14 which extends the entire length of the same. In some cases it may be necessary to excavate the upper most portion of the canal 38 to facilitate insertion of the wedge member 32 and accommodate the wider upper portion 20 of the wedge member 32.

The surgeon then replaces the head of the femur 14, i.e., ball, and the acetabulum, i.e., socket, with new, biocompatible artificial parts, e.g., ball 18 and socket 16, made of materials which permit a natural, gliding motion of the hip joint, e.g., cobalt-chromium-molybdenum and/or titanium-based alloys. In some cases the surgeon uses a special glue or cement to bond the new parts of the hip joint to the existing healthy bone 14. In other cases the artificial parts are made from or include a porous biocompatible material which permits the patient's own bone to grow into the pores and hold the new parts in place.

It has been seen, however, that with prostheses that have been cemented in place the various stress loads, i.e., the compression, shear and torsion, to which the implanted device is normally subjected may cause the bone-cement bond to weaken. Other factors such as osteoporosis, also tend to weaken the bone cement bond.

The various embodiments of the present disclosure provide configurations which cooperate with ultrasonic therapies to induce bony ingrowth into the prosthetic device and provide a more stable fixation between the prosthesis and the bone.

As illustrated in Figs. 1A-1C, once the prosthesis has been properly implanted, the upper portion 20 of the wedge member 32 protrudes from the upper portion of the femur 14 to expose an opening 22 disposed proximate the uppermost portion 20 of the wedge member 32. This opening 22 leads to a resonating chamber 34 which extends inwardly down the wedge member 32 towards the tapered end portion 36 as best seen in Fig. 1C.

An external ultrasonic transducer 12 is applied to the outer skin of the patient (preferably pre-treated with a lotion or gel specifically developed to reduce the chances of the skin developing rashes or burning) and emits acoustic waves 30 at frequencies of between about 5KHz to about 10kHz which are transcutaneously delivered through the body tissue and muscle 28, 32 towards the upper portion 20 of the prosthesis 10. Preferably, the acoustic wave 30 is focused at opening 22 such that the majority of the wave 30 enters through the opening 22 and into the resonating chamber 34 which is filled with a fluid to facilitate propagation of the acoustic wave 30' through the resonating chamber.

As best seen in Figs. 1B and 1C, the uppermost portion of the resonating chamber 34 is equipped with a reflective surface 42 which is specifically configured and dimensioned to reflect waves 30 downwardly through the resonating chamber 34. Preferably, the reflected waves 30' resound off the interior walls of the resonating chamber 34 and cause the prosthesis 10 to resonate.

By resonating the prosthesis 10, or a portion thereof, within the medullary canal 38 of the femur 14 at specific frequencies for short, e.g., 20 minute, periods of time on a weekly, bi-weekly, daily, or other time-specific basis, the energy will stimulate the soft cancellous bone 40 which surrounds the medullary canal 38 to grow inwardly, i.e., "bony ingrowth", and stabilize the prosthesis 10 within the femur 14.

It is contemplated that the cross-sectional areas of the resonating chamber 34 can be varied along the length thereof to adjust the depth of penetration of the propagated wave. In addition, the excitation values can be varied to promote bony ingrowth from the distal end 36 to the upper portion 20.

Preferably, the reflective surface 42 can be configured at any desired angle to resound acoustic waves 30' downward through the resonating chamber 34 to cause the prosthesis to resonate/vibrate at different frequencies. Although it is preferable to utilize a resonating chamber 34 which has a natural resonance which responds to the acoustic waves 30' being propagated therethrough, in some cases it may be desirable to configure the resonating chamber 34 to have a more cylindrical-like cross-section or some other geometrically advantageous cross-section to produce a different or specific desired resonating effect.

In some cases it may also be preferable to calibrate the transducer 12 to emit one steady frequency to resonate the prosthesis 10, or in other cases it may be preferable to sweep the modulating frequency across a wide range of frequencies to stimulate bony ingrowth. See, e.g., U.S. Patent No. 5,520,612, incorporated herein by reference.

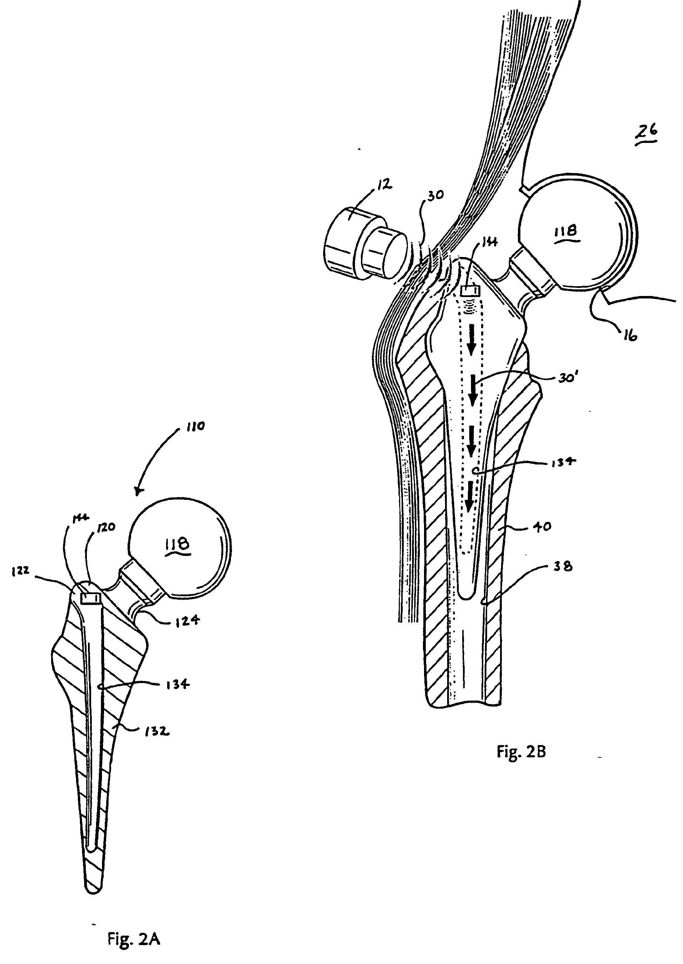

Figs. 2A and 2B show an alternate embodiment of the present disclosure which include a hip prosthesis 110 generally configured, dimensioned and operable in the same fashion as the Figs. 1A-1C embodiment with the exception that this embodiment includes a second acoustically coupled transducer 144 disposed internally within the upper portion 120 of the wedge member 132 of the prosthesis 110. In use, the external transducer 12 emits acoustic energy (focused or unfocused) at the second transducer 144 which, in turn, emits acoustic waves 30' downward through the resonating chamber 134. Preferably, the dimensions of the second transducer 144 are matched to the frequency to facilitate wave 30' propagation through the fluid-filled resonating chamber 134 and/or the Eigen modes are matched to the cavity.

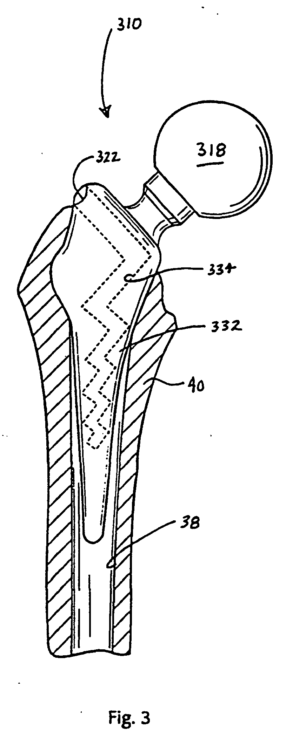

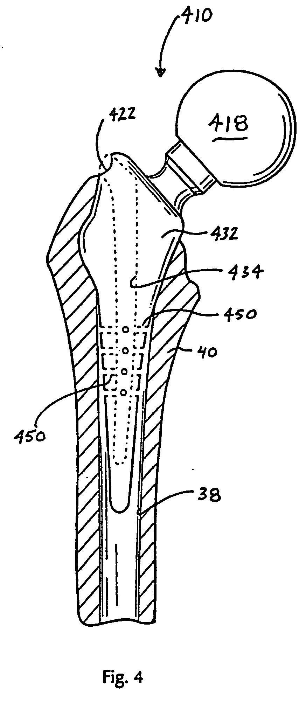

Figs. 3 and 4 show alternate configurations of the resonating chamber 334, 434 disposed within the wedge member 332, 432 of the hip prosthesis 310, 410. More particularly, Fig. 3 shows a zig-zag-like resonating chamber 334 internally disposed within the wedge member 332. It is believed that configuring the chamber 334 in this fashion will enhance vibration of the prosthesis which will, in turn, further stimulate bony ingrowth of the soft cancellous bone 40 surrounding the medullary canal 38.

Fig. 4 shows another alternate configuration of the resonating chamber 434 wherein a series of generally laterally disposed slots 450 extend from the interior walls of the resonating chamber 434 to the outermost periphery of the wedge member 432 to propagate the acoustic energy 30' directly through the prosthesis 410 to the surrounding soft cancellous bone 40 of the medullary canal 38. It is further contemplated that structures such as biocompatible ball bearings, blades, wires, etc. can be positioned within the slots 450 to enhance the propagation of energy to the surrounding bone 40.

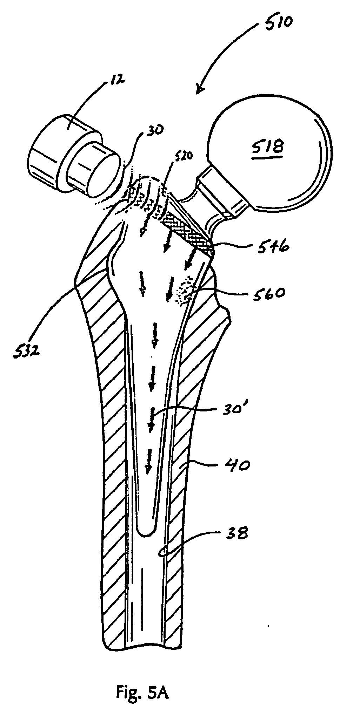

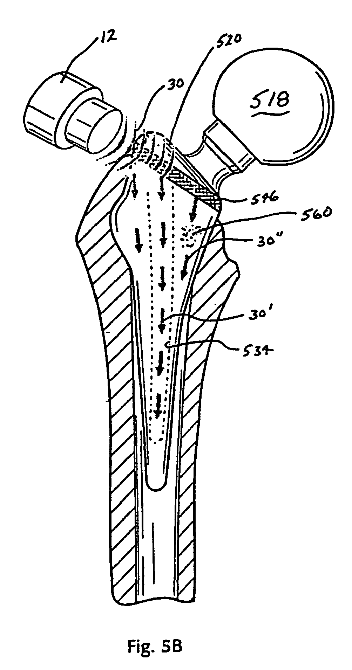

Fig. 5A and 5B show two additional alternate embodiments of the present disclosure incorporating a transducer collar 546 which transfers/emits acoustic waves 30' downward along the outer periphery of the wedge member 532 to stimulate bony ingrowth. More particularly and with reference to Fig. 5A. the hip prosthesis 510 of this embodiment includes a generally circular transducer collar 546 which surrounds the uppermost portion 520 of the wedge member 532. Collar 546 is activated and/or energized by the acoustic waves 30 emitted from external transducer 12 and, in turn, propagates waves 30' downwardly along the outer periphery of the wedge member 532 to stimulate ingrowth of the soft cancellous bone 40 surrounding the medullary canal.

Preferably, collar 546 can work in combination with other wave propagation devices. For example, collar 546 can work in combination with a porous material 560 which wraps the wedge member 532 and helps to propagate the acoustic wave 30' downwardly to stimulate the soft bone 40. More particularly, the acoustic wave 30' is trapped within the porous layer 560 as it travels/conducts downward through the wedge member 532 and/or the porous layer 560 acts as its own waveguide. The medullary canal of this embodiment may act as a type of waveguide further enhancing/stimulating bony ingrowth.

It is also contemplated to impinge the acoustic wave 30 from the external transducer 12 directly upon the porous material 560 above the femur 14 such that the acoustic wave 30 travels within the porous layer 560 downward along the outer periphery of the wedge member 532 without the use of collar 546.

Fig. 5B shows an alternate embodiment of the Fig. 5A embodiment wherein the wedge member 532 also includes an internally disposed resonating chamber 534 which is configured and dimensioned similar to the resonating chamber of Fig. 3 to propagate waves 30' internally through the wedge member 532. As shown in this figure, acoustic waves 30" and 30' are propagated along the outer periphery of the wedge member 532 and within the resonating chamber 534, respectively, which, it is contemplated, will have a duel effect of enhancing bony ingrowth.

Although Fig. 5B shows the resonating chamber similar to Fig. 3, it is contemplated that other embodiments of the resonating chamber described herein may be used in combination with the transducing collar 546 to enhance bony ingrowth.

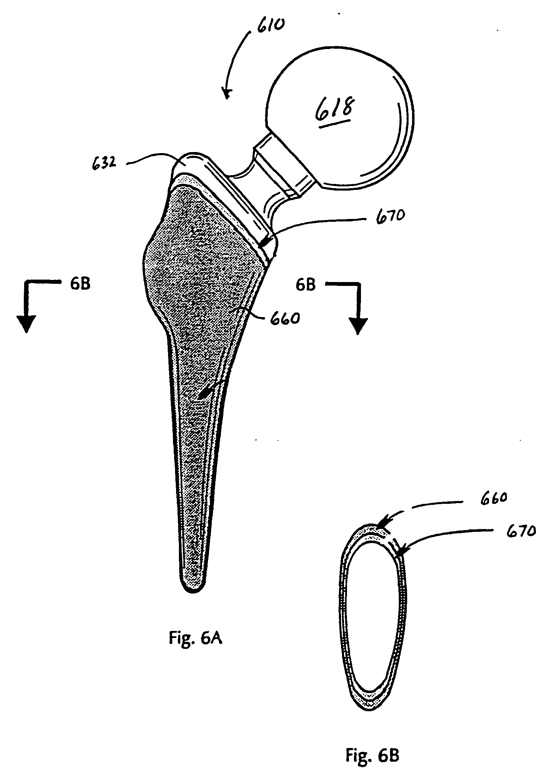

Figs. 6A and 6B show yet another alternate embodiment of the hip prosthesis 610 which includes a piezoelectric/piezoceramic membrane material 670 disposed between the wedge member 632 and the porous coating 660. Preferably, the piezoelectric/piezoceramic material is activated externally, e.g., by external transducer 12, and operates to propagate acoustic energy downward along the outer periphery of the wedge member 632 to stimulate bony ingrowth from the soft bone 40 into the porous coating to stabilize the prosthesis 610.

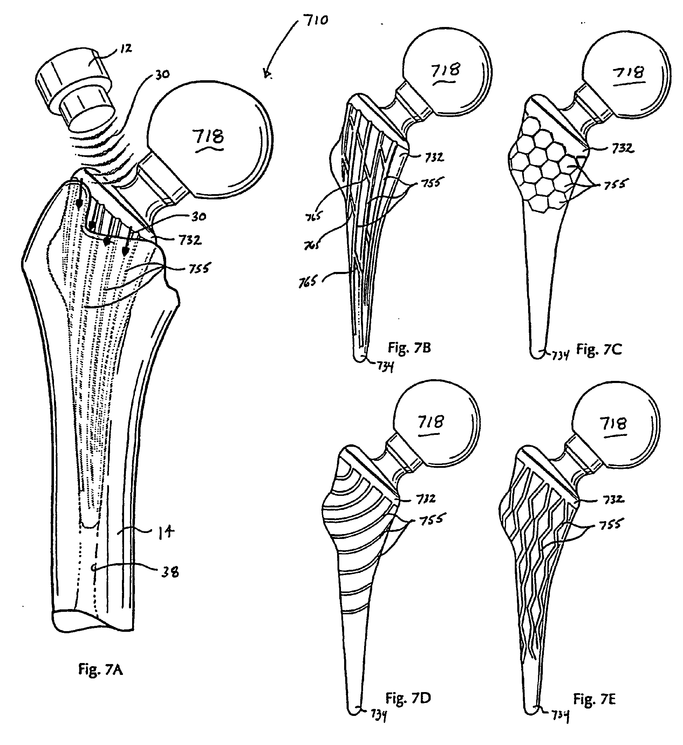

Figs. 7A-7E show alternate embodiments of hip prosthesis wherein the outer periphery of the wedge member 732 is patterned to conduct/transmit acoustic waves 30 directly into the medullary canal 38. For example; the different patterns of the wedge member 732 can include grooves or channels (Figs. 7A and 7B), honeycomb patterns (Fig. 7C), semi-circular/spiral groove patterns (Fig. 7D) and/or a series of longitudinally or laterally oriented zig-zag patterns (Fig. 7E). These patterns have a two-fold effect: 1) to directly conduct acoustic waves 30 into the medullary canal 38 to stimulate bony ingrowth; and 2) to enhance the fit of the prosthesis 710 in the medullary canal 38 during implantation.

In use and as best seen in Fig. 7A, an external transducer 12 emits acoustic waves 30 towards the upper portion 720 of the wedge member 732. The acoustic waves 30, in turn, travel along the outer periphery of the wedge member 732 and are trapped within the specified pattern thus propagating the waves 30 within the pattern between the wedge member 732 and the medullary canal. It is contemplated that these patterns promote better ultrasound coverage and, thus, enhance the coverage of bony ingrowth.

Fig. 7B shows one particular embodiment of the present disclosure which includes a vertical groove pattern 755 which extends along the outer periphery of the prosthesis 710 to conduct acoustic waves 30 into the medullary canal 38. This particular embodiment also includes a series of downwardly angled bridges 765 disposed between adjacent grooves 755 which provide alternate paths for the acoustic energy 30 should a groove 755 become saturated with bony ingrowth.

For the purpose of analysis, the etched patterns/grooves 755 on the outer surface of the prosthesis 710 can be considered to be a unique collection of rectangular waveguides, each of dimensions $d_x$ and $d_y$ and open in the z-direction. If a particular choice of "n" and "m" specifies one of the possible normal modes of vibration, then the cutoff frequency ($f_c$) for the nm$^{th}$ mode is:

$$f_c = (\frac{c_L}{2})\left[(\frac{n}{d_x})^2 + (\frac{m}{d_y})^2\right]^{\frac{1}{2}}$$

For n=m=1, the longitudinal velocity of sound $c_L$ = 1500 meters per second, then the cutoff frequencies for the channels are:

$$d_x = d_y = 6mm, f_c \geq 177 \text{ kHz}; \quad d_x = d_y = 3mm, f_c \geq 354 \text{ kHz}; \quad d_x = d_y = 1.5mm, f_c \geq 707 \text{ kHz}.$$

Insonification of the channels 755 at frequencies much lower than $f_c$ will produce vibrational modes of primarily shear waves.

Since the femur is generally cylindrical and about one-forth of the body weight and if the channel 755 is considered to be a about two-thirds of the femur length and if the channel 755 is filled with body fluid, blood, and some tissue debris, then the ultrasound absorption can be assumed to be about 0.3 dB per MHz per cm. For example, the femur of a person who is six feet tall is about eighteen inches in length, thus, the prosthesis 710 should have a grooved channel 755 about nine inches (23 cm) in length. If the transmitted frequency is 1.0 MHz, then the maximum absorption incurred for this channel size is about 7dB, or an 80% reduction in acoustic intensity from the proximal to distal end. The acoustic power is variable to ensure sufficient spatial average-temporal average (SATA) intensity levels along the channel 755 to induce bony tissue ingrowth through ultrasound stimulation.

If the conventional porous coatings on the prosthesis are distributed at strategic locations, primarily at the proximal end, an acoustic mode can be produced to generate shear waves at these specific locations to enhance bony tissue ingrowth - a type of induced "spot tissue-welding".

Preferably, the frequency of wave 30 and the channel/groove 755 sizes are varied to promote bony ingrowth from the distal to proximal ends of the prosthesis. It is contemplated that by promoting bony growth in this fashion, the entire prosthesis 710 can fuse within the medullary canal 38 of the bone.

Alternatively, the wedge member 732 can also include an internally disposed resonating chamber which can be configured and dimensioned similar to the resonating chamber of Fig. 1 to propagate waves internally through the wedge member 732. As such, acoustic waves can be propagated along the outer periphery of the wedge member 732 and within the resonating chamber, respectively, to enhance the bony ingrowth.

Although the channels 755 shown herein are shown to have a U-shaped cross-section, it is envisioned that other shapes can be used to which may promote enhanced fusion of the prosthesis 710 with the bony tissue ingrowth, e.g., undercut, rectangular and/or hemispherical.

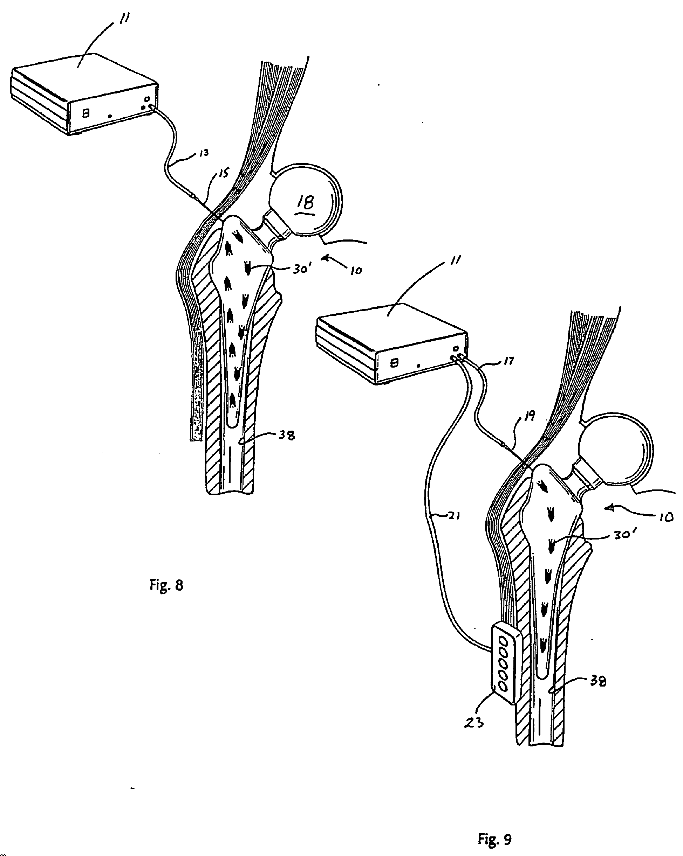

Figs. 8 and 9 illustrate a reflected diagnostic system for determining, e.g., whether any one of the aforedescribed ultrasonic therapies are required, i.e., the prosthesis has loosened with the medullary canal. More particularly, Fig. 8 shows a main unit 11 equipped with a send/receive probe 15 which is placed in contact with the prosthesis 10. A signal is applied from unit 11 through cable 13 to probe 15 and to prosthesis 10. The return signal from the prosthesis is received by the receive portion of the probe after the signal propagates/travels through the prosthesis. Preferably, the main unit 11 includes a learned neural net which compares prior data and actual live data to determine prognosis, i.e., the return signal is analyzed and compared to prior acoustic/signal data taken at the time of implantation or last treatment to determine if the prosthesis 10 has loosened and/or the extent of bony ingrowth.

Fig. 9 shows an alternative diagnostic system wherein a second needle and/or sensor array 23 is placed adjacent the bottom of the prosthesis 10 to directly receive the primary signal from the main unit 11 at predetermined points along the prosthesis. The signal is then analyzed and compared to prior acoustic/signal data taken at the time of implantation or last treatment to determine if the prosthesis 10 has loosened. Preferably, the sensor array 23 will give progressive readings and relay the information back to the main unit 11 via cable 21.

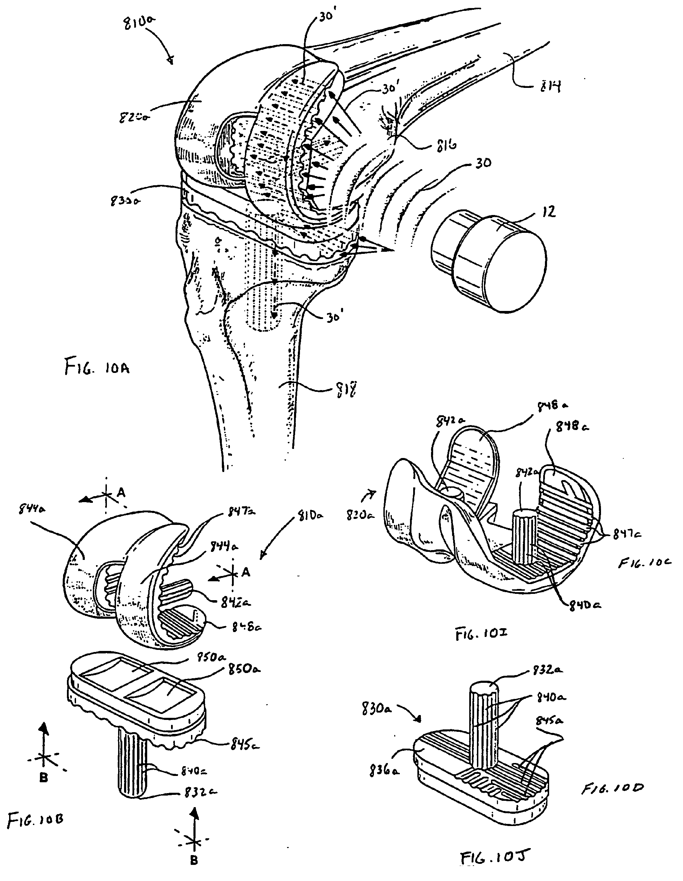

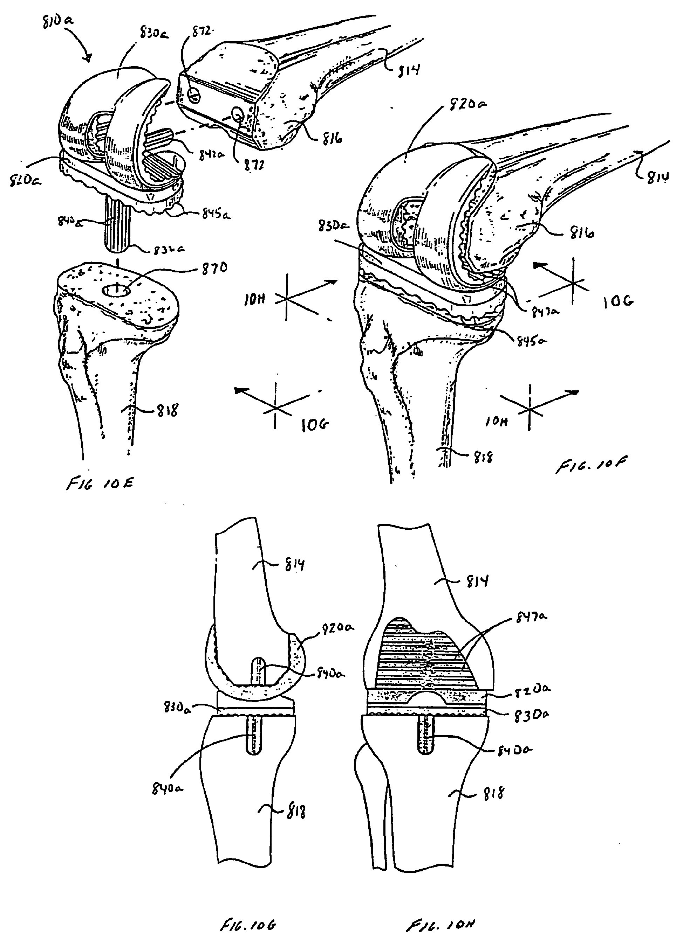

Figs. 10A-10J show an alternate embodiment of the present disclosure which includes a knee joint prosthesis 810a having an upper prosthetic implant 820a and a lower prosthetic implant 830a which are designed to engage one another to form the prosthetic joint. Upper implant 820a is generally U-shaped and dimensioned to receive and encompass the patella 816 of the femur 814. Lower implant 830a is generally T-shaped and dimensioned to fit atop the distal end of the tibia 818. During knee joint replacement surgery, the surgeon removes the diseased portion of the bone, surrounding tissue and cartilage from the joint and reshapes the patella 816 and the distal end of the tibia 818 to receive upper and lower implants 820a and 830a, respectively (see Fig. 10E).

Preferably, the upper implant includes a pair of dowels 842a which project from the implant 820a and are generally dimensioned to engage a corresponding pair of bores 872 which are drilled into the patella 816. Likewise, the lower implant 830a includes a dowel 832a which engages a corresponding bore 870 which is drilled into the distal end of the tibia 818. Preferably, the dowels 842a and 832a include a plurality of channels or grooves 840a which extend along the length of the dowels 842a and 832a and which provide a dual function: 1) facilitate insertion and stability of the dowels within bores 870 and 872; and 2) provide a pathway to propagate acoustic energy 30' into the bone 816, 818 to stimulate bony ingrowth. In some cases the surgeon can use a special glue or cement to bond the implants 820a, 830a to the existing healthy bone 816, 818. In other cases the implants 820a, 820b can include a porous biocompatible material which permits the patient's own bone to grow into the pores and hold the implants 820a. 830a in place.

Preferably, the inner periphery of both the upper and lower implants 820a, 830a also include a plurality of grooves 847a and 845a, respectively, which also stabilize the upper and lower implants 820a, 830a atop the bone 816, 818 and provide a pathway for the acoustic energy 30' to stimulate bony ingrowth.

As seen best in Fig. 10B, the top portion of the lower implant 830a includes a pair of rectilinear recesses 850a which are designed to seat the lowermost portion of the upper implant 820a in a cradle-like manner. This permits a natural, rocking motion of the implants 820a, 830a relative to one another which mimics the natural motion of the original knee joint. Once the upper and lower implants are engaged, the muscles and ligaments which surround the knee joint are replaced and cooperate to retain the joint 810a in place.

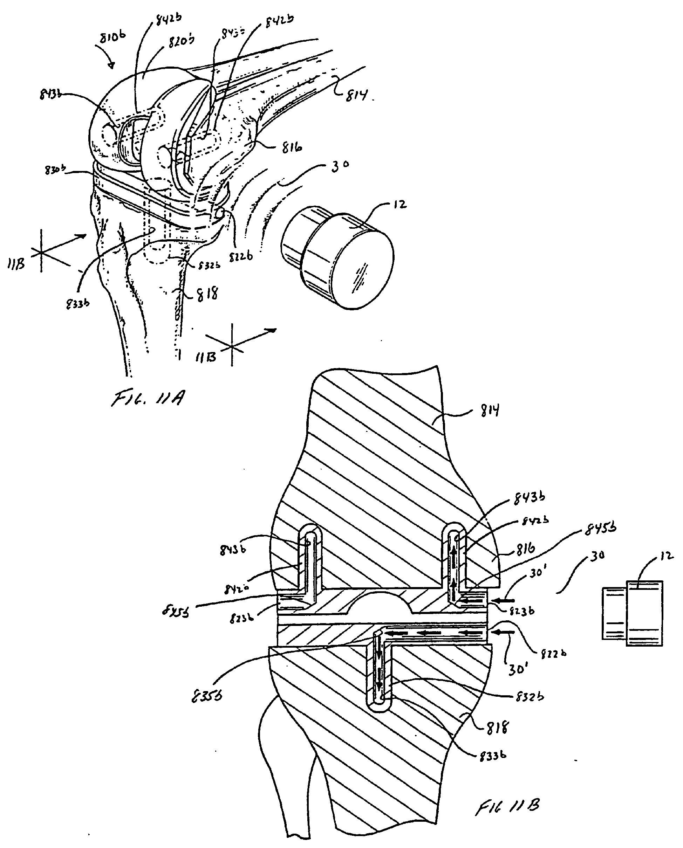

Figs. 11A and 11B show an alternate embodiment of a knee prosthesis of the present disclosure which includes similar upper and lower implants 820b and 830b which engage one another in a similar cradle-like fashion to form the prosthetic knee joint 810b. More particularly, upper and lower implants 820b, 830b are generally shaped to engage the patella 816 and distal end of the tibia 818, respectively, in a similar manner as described above with respect to the Figs. 10A-10H embodiment with the exception that instead of grooves, each dowel 842b and 832b includes an elongated resonating chamber 843b and 833b, respectively, which extends the length thereof.

As seen best in Fig. 11B, upper and lower implants 820b and 830b also include side channels 823b and 822b, respectively, which carry the acoustic energy 30' toward the resonating chambers 843b, 833b of the dowels 842b, 832b. Preferably, each side channel 823b, 822b also includes a reflective surface 845b, 835b, respectively, which directs the acoustic energy 30' into the corresponding resonating chambers 843b, 833b. Preferably, the reflected waves 30' resound off the interior walls of the resonating chambers 843b, 833b and cause the prosthesis 810b to resonate which stimulates bony ingrowth.

By resonating the prosthesis 810a or 810b, or a portion thereof, within the patella 816 and tibia 818 at specific frequencies for short, e.g., 20 minute, periods of time on a weekly, bi-weekly, daily, or other time-specific basis, the energy will stimulate the soft cancellous bone which surrounds the dowels 842a,b and 832a,b to grow inwardly and stabilize the upper and lower members 820a,b and 830a,b.

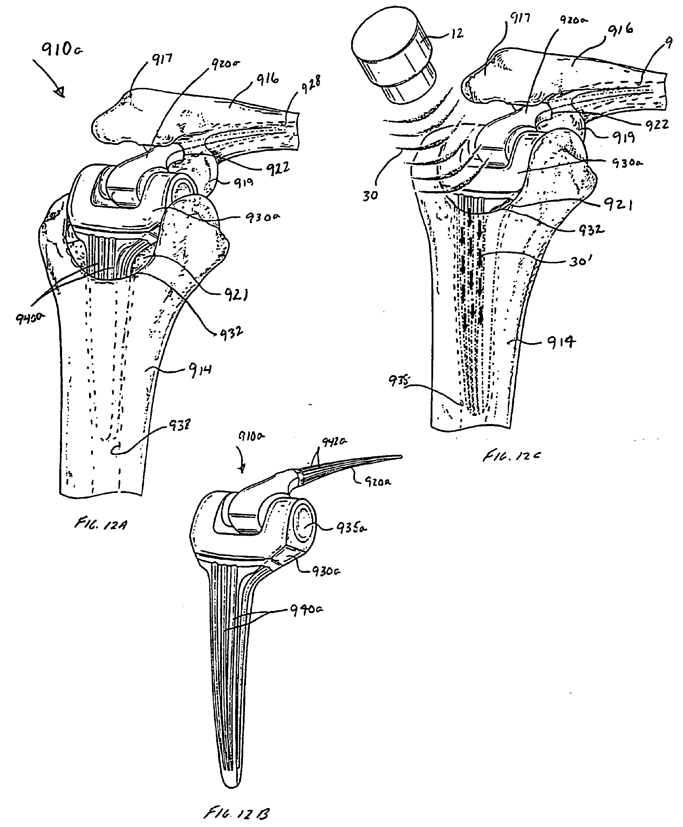

Figs. 12A-12C show yet another embodiment of the present disclosure which includes an elbow prosthetic implant 910a designed to engage the lower end of the humerus 914 and upper end of the ulna 916. More particularly, the prosthetic device 910a includes a pair of tapered, spike-like insertion members 920a and 930a which are pivotally joined to one another about a pivot 935a. Upper spike member 920a is dimensioned to insert into bore 922 which is drilled into the medullary canal 928 of the ulna 916 preferably between the olecranon process and the coronoid process. Lower spike member 930a is dimensioned to insert into bore 932 which is drilled through the olecranon depression 21 and into the medullary canal 938 of the humerus. In much the same manner as described with the above prosthetic devices, the surgeon can use special glues or cement to bond the spike members 920a, 930a to the existing healthy bone 916, 918 or porous materials which permit the patient's own bone to grow into the pores and hold the spike members 920a, 930a in place.

Preferably the outer periphery of each of the spike members 920a, 930a includes a plurality of grooves or channels 942a and 940a, respectively, which facilitate insertion and stabilization of spike members 920a and 930a within bores 928, 938 and also provide pathways for the propagation of acoustic energy 30' into bones 816, 818 to stimulate bony ingrowth. In use and as seen best in Fig. 12C, an external transducer 12 emits acoustic waves 30 towards the lower spike member 930a. The acoustic energy 30', in turn, travels along the outer periphery of the spike member 930a between the spike member 930a and the medullary canal 938 to stimulate bony ingrowth.

As mentioned above, the spike members 920a, 930a are joined to one another by pivot 935a which permits natural, pivotal motion of the spike members 920a, 930a relative to one another to mimic the natural motion of the original elbow joint.

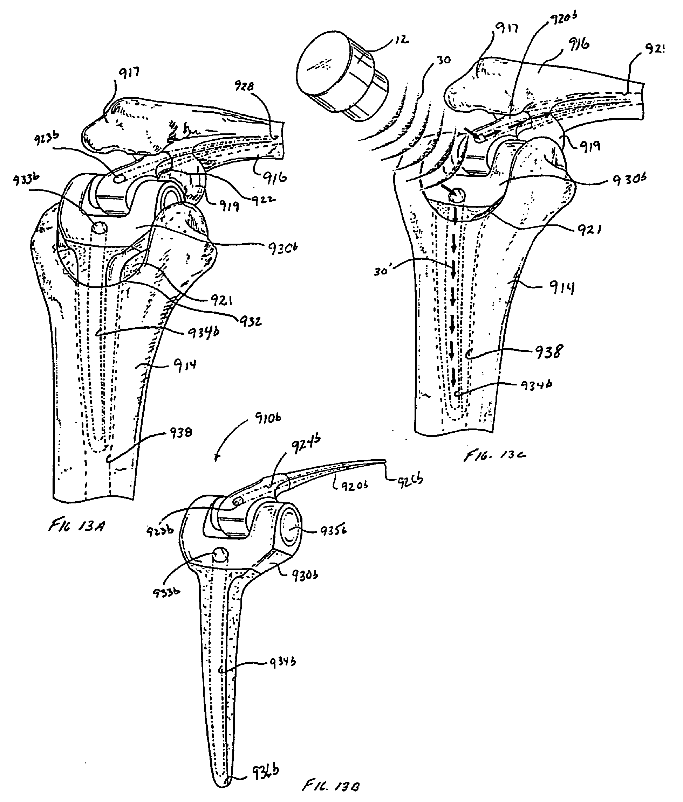

Figs. 13A-13C show an alternate embodiment of an elbow prosthesis 910b according to the present disclosure which includes similar upper and lower spike members 920b and 930b which are joined about pivot 935b to form the prosthesis 910b. Much like the embodiment shown in Figs. 12A-12C, spike members 920b and 930b are shaped for insertion into corresponding bores 922, 932, respectively, drilled into the medullary canals 928, 938 of the ulna 916 and the humerus 914. However, instead of grooves disposed along the outer periphery of the spike members 920b, 930b, each spike member 920b, 930b includes an elongated resonating chamber 924b and 934b, respectively, which extend the length thereof towards respective distal ends 926b and 936b.

As seen best in Fig. 13C, external ultrasonic transducer 12 emits acoustic waves 30 which are transcutaneously delivered through the body tissue towards the prosthesis 910b. Preferably, the acoustic wave 30 is focused at openings 923b, 933b such that a majority of the wave energy enters openings 923b, 933b of resonating chambers 924b, 934b and resounds off the interior walls of resonating chamber 9244b, 934b and causes each spike member 920b, 930b to resonate. In some cases it may be preferable to fill resonating chamber 924b, 934b with a fluid to facilitate propagation of the acoustic wave 30' through the resonating chambers 924b, 934b.

By resonating the spike members 920b, 930b within the medullary canals 928, 938, the resounding energy will stimulate the soft cancellous bone which surrounds the canals 928, 938 to grow inwardly and stabilize the prosthesis 910b within the bones 916, 914.

From the foregoing and with reference to the various figure drawings, those skilled in the art will appreciate that certain modifications can also be made to the present disclosure without departing from the scope of the present disclosure. For example, in some case it may be preferable to employ a converter to convert ultrasound to the power transducer and/or use an audio signal to activate an internally housed transducer. Although it is preferable to fill the resonating chamber with a ultrasound conducting fluid, in some cases it may be preferable to shape the resonating chamber as a resonating horn filled with a solid material.

Although the various prosthetic devices of Figs. 10-13 show resonating chambers similar in construction to the resonating chamber of Fig. 3, it is contemplated that various resonating chambers described herein may be used in combination with the prosthetic devices of the Figs. 10-13 embodiments. Likewise, it is contemplated that the various patterns shown on the outer periphery of the prostheses shown with respect to Figs. 7A-7E can be employed on the prosthetic devices shown in Figs. 10-13.

In addition to the internal and external waveguides shown herein, it is envisioned that the structure can be used in conjunction with porous coatings of the type known in the art either arranged in predetermined patterns or uniformly, see, e.g., U.S. Patent No. 4,536,854, U.S. Patent No. 5,018,285 and U.S. Patent No. 5,004,476.

The various embodiments of the present disclosure provide configurations which cooperate with ultrasonic therapies to induce bony ingrowth into the prosthetic device and provide a more stable fixation between the prosthesis and the bone. While particular embodiments of the disclosure have been described, it is not intended that the disclosure be limited thereto, as it is intended that the disclosure be as broad in scope as the art will allow and that the specification be read likewise. Therefore, the above description should not be construed as limiting, but merely as exemplifications of preferred embodiments. Those skilled in the art will envision other modifications within the scope of the present disclosure.

**Claims**

1. A bone prosthesis (10) comprising a first portion (32) for insertion into a medullary canal of or a bore in a first bone segment (14) said part including an internal channel, said internal channel having an interior reflective surface (42) which defines a resonating chamber (34) disposed through said part for propagating acoustic energy (30) to the medullory canal of or bore in said first bone segment to stimulate growth of bone in said medullary canal or bore adjacent the part and thereby stabilise the part in said medullary canal or bore, **characterised in that** the outer periphery of the first portion is patterned to promote acoustic wave propagation along its outer surface.

2. A bone prosthesis (10) according to claim 1, wherein said resonating chamber (34) includes at least one opening for receiving acoustic energy (30).

3. A bone prosthesis (10) according to claim 1 or 2,wherein said resonating chamber (34) is convoluted.

4. A bone prosthesis (10) according to any one of claims 1 to 3, which includes a transducer (144) for receiving acoustic energy (32) and emitting acoustic waves (30) through said channel.

5. A bone prosthesis (10) according to claim 4, wherein the transducer (144) is a transducer collar (546).

6. A bone prosthesis (10) according to claim 4, wherein the transducer (144) is disposed in the channel.

7. A bone prosthesis (10) according to any one of the preceding claims, wherein a porous coating is provided on the part.

8. A bone prosthesis (10) according to claim 7, wherein a piezoelectric membrane material is disposed between said porous coating and an outer periphery of said part.

9. A bone prosthesis (10) according to claim 7, wherein a piezoceramic membrane material is disposed between said porous material and an outer periphery of said part.

10. A bone prosthesis (10) according to any one of the preceding claims, which includes a ball portion for engaging the acetabulum of the pelvic bone (26) and said part of the first portion is an implant for engaging the medullary canal (38) of the femur (14).

11. A bone prosthesis (10) as claimed in claim 1 in which the pattern is a zig-zag pattern.

**12.** A bone prosthesis (10) according to claim 10, wherein said resonating chamber (34) includes a plurality of slots which extend outwardly from said resonating chamber (34) to transmit acoustic energy (30) directly to the medullary canal (38).

**13.** A bone prosthesis (10) according to claim1, further comprising a second portion having a part for insertion into medulary canal or bore in a second bone segment.

**14.** A bone prosthesis (10) according to claim 13, wherein said part of the second portion includes at least one channel for propagating acoustic energy there through to the second bone segment to stimulate growth of bone in said medullary canal (38) or bore adjacent said part and thereby stabilise said part in the medullary canal (38) of or bore in the second bone segment.

**15.** A bone prosthesis (10) according to claim 14 wherein said at least one channel is an internal channel having an interior reflective surface (42) which defines a resonating chamber (34) disposed through said part of the second portion.

**16.** A bone prosthesis (10) according to claim 15 wherein said resonating chamber (34) includes at least one opening for receiving acoustic energy.

**17.** A bone prosthesis (10) according to claim 13 wherein one of the first and second portions is adapted to engage the medullary canal (38) of the humerous and the other of the first and second portions is adapted to engage the medullary canal (38) of the ulna, said first and second portions being movable relative to each other about a pivot.

**18.** A bone prosthesis (10) according to claim 17 wherein an outer periphery of said part of the second portion is patterned to promote acoustic wave propagation along an outer surface of said part.

**19.** A bone prosthesis (10) according to claim 13, wherein one of the first and second portions is adapted to engage the femur (14) and other of the first and second portions adapted to engage the tibia (818), said first and second portions being movable relative to one another.

**20.** A bone prosthesis (10) according to claim 19, wherein said part of the one portion comprises a dowel for insertion in a corresponding bore associated with the femur (14) and said part of the other portion comprises a dowel for insertion in a corresponding bore associated with the tibia (818).

**21.** A bone prosthesis (10) according to claim 19 or 20, wherein an outer periphery of said part of the second portion is patterned to promote acoustic wave propagation along an outer surface of said part.

**22.** A bone prosthesis (10) according to claim 20 or 21, wherein one or both of the first and second portions includes a further dowel for insertion in a corresponding bore associated with the respective bone segment.

**23.** A bone prosthesis (10) according to claim 22, wherein said further dowel includes at least one channel for propagating acoustic energy (30) therethrough to the respective bone segment to stimulate growth of bone in said corresponding bore adjacent said further dowel and thereby stabilise said further dowel insaid corresponding bore.

**24.** A bone prosthesis (10) according to claim 23, wherein said at least one channel is an internal channel having an interior reflective surface (42) which defines a resonating chamber (34) disposed through said further dowel.

**25.** A bone prosthesis (10) according to claim 24, wherein said resonating chamber (34) includes at least one aperture for receiving acoustic energy (30).

**26.** A bone prosthesis (10) according to any one of claims 23 to 25, wherein a plurality of surface grooves are provided on the further dowel for propagating acoustic energy (30) through the grooves.

**27.** A bone prosthesis (10) according to claim 19, wherein said one and other of the first and second portions include outer surfaces which povitally engage one another and bone-facing inner surfaces which are adapted to engage the femur (14) and tibia (818), respectively.

**28.** A bone prosthesis (10) according to claim 27, wherein said one portion is generally U-shaped and is adapted to encompass the patella of the femur (14) and said other portion is generally T-shaped and is adapted to fit atop the tibia (818).

29. A bone prosthesis (10) according to claim 27 wherein said one and other of the first and second portions include a plurality of grooves located along said bone-facing inner surface thereof.

30. A bone prosthesis (10) according to claim 27, wherein said outer surface of said other portion includes at least one recess for seating the outer surface of said one portion in a cradle-like manner.

31. A bone prosthesis (10) as claimed in claim 1 in which the pattern is a vertical groove pattern.

32. A bone prosthesis (10) as claimed in claim 1 in which the pattern is a honey comb pattern.

33. A bone prosthesis (10) as claimed in claim 1 in which the pattern is a semi-circular/spiral pattern.

**Patentansprüche**

1. Eine Knochenprothese (10), die einen ersten Abschnitt (32) zum Einsetzen in einen Markkanal eines ersten Knochensegment (14) oder in eine Bohrung in einem ersten Knochensegment beinhaltet, wobei der Teil einen internen Kanal umfasst, wobei der interne Kanal eine innere reflektierende Oberfläche (42) aufweist, die eine Resonanzkammer (34) definiert, welche durch den Teil angeordnet ist, um akustische Energie (30) zum Markkanal des ersten Knochensegments oder zu der Bohrung in dem ersten Knochensegment weiterzuleiten, um das Wachstum von Knochen in dem Markkanal oder der Bohrung neben dem Teil zu stimulieren und **dadurch** den Teil in dem Markkanal oder in der Bohrung zu stabilisieren, **dadurch gekennzeichnet, dass** der Außenumfang des ersten Abschnitts gemustert ist, um eine Weiterleitung von akustischen Wellen entlang seiner Außenoberfläche zu fördern.

2. Knochenprothese (10) gemäß Anspruch 1, wobei die Resonanzkammer (34) mindestens eine Öffnung zum Empfangen von akustischer Energie (30) umfasst.

3. Knochenprothese (10) gemäß Anspruch 1 oder 2, wobei die Resonanzkammer (34) gewunden ist.

4. Knochenprothese (10) gemäß einem der Ansprüche 1 bis 3, die einen Wandler (144) zum Empfangen von akustischer Energie (32) und Ausgeben von akustischen Wellen (30) durch den Kanal umfasst.

5. Knochenprothese (10) gemäß Anspruch 4, wobei der Wandler (144) ein Wandlerring (546) ist.

6. Knochenprothese (10) gemäß Anspruch 4, wobei der Wandler (144) in dem Kanal angeordnet ist.

7. Knochenprothese (10) gemäß einem der vorhergehenden Ansprüche, wobei auf dem Teil eine poröse Beschichtung bereitgestellt ist.

8. Knochenprothese (10) gemäß Anspruch 7, wobei ein piezoelektrisches Membranmaterial zwischen der porösen Beschichtung und einem Außenumfang des Teils angeordnet ist.

9. Knochenprothese (10) gemäß Anspruch 7, wobei ein piezokeramisches Membranmaterial zwischen dem porösen Material und einem Außenumfang des Teils angeordnet ist.

10. Knochenprothese (10) gemäß einem der vorhergehenden Ansprüche, die einen Kugelabschnitt zum Eingreifen in das Acetabulum des Hüftknochens (26) umfasst, und der Teil des ersten Abschnitts ist ein Implantat zum Eingreifen in den Markkanal (38) des Femurs (14).

11. Knochenprothese (10) gemäß Anspruch 1, in der das Muster ein Zick-Zack-Muster ist.

12. Knochenprothese (10) gemäß Anspruch 10, wobei die Resonanzkammer (34) eine Vielzahl von Schlitzen umfasst, die sich von der Resonanzkammer (34) nach außen erstrecken, um akustische Energie (30) direkt auf den Markkanal (38) zu übertragen.

13. Knochenprothese (10) gemäß Anspruch 1, die ferner einen zweiten Abschnitt mit einem Teil zum Einsetzen in einen Markkanal oder in eine Bohrung in einem zweiten Knochensegment beinhaltet.

14. Knochenprothese (10) gemäß Anspruch 13, wobei der Teil des zweiten Abschnitts mindestens einen Kanal umfasst, um akustische Energie dahindurch zum zweiten Knochensegment weiterzuleiten, um das Wachstum von Knochen in dem Markkanal (38) oder der Bohrung neben dem Teil zu stimulieren und **dadurch** den Teil in dem Markkanal (38) des zweiten Knochensegments oder in der Bohrung in dem zweiten Knochensegment zu stabilisieren.

15. Knochenprothese (10) gemäß Anspruch 14, wobei der mindestens eine Kanal ein interner Kanal mit einer inneren reflektierenden Oberfläche (42) ist, die eine Resonanzkammer (34) definiert, welche durch den Teil des zweiten Abschnitts angeordnet ist.

16. Knochenprothese (10) gemäß Anspruch 15, wobei die Resonanzkammer (34) mindestens eine Öffnung zum Empfangen von akustischer Energie umfasst.

17. Knochenprothese (10) gemäß Anspruch 13, wobei einer von dem ersten und dem zweiten Abschnitt angepasst ist, um in den Markkanal (38) des Humerus einzugreifen, und der andere von dem ersten und dem zweiten Abschnitt angepasst ist, um in den Markkanal (38) der Ulna einzugreifen, wobei der erste und der zweite Abschnitt relativ zu einander um einen Drehpunkt bewegbar sind.

18. Knochenprothese (10) gemäß Anspruch 17, wobei ein Außenumfang des Teils des zweiten Abschnitts gemustert ist, um eine Weiterleitung von akustischen Wellen entlang einer Außenoberfläche des Teils zu fördern.

19. Knochenprothese (10) gemäß Anspruch 13, wobei einer von dem ersten und dem zweiten Abschnitt angepasst ist, um in den Femur (14) einzugreifen, und der andere von dem ersten und dem zweiten Abschnitt angepasst ist, um in die Tibia (818) einzugreifen, wobei der erste und der zweite Abschnitt relativ zu einander bewegbar sind.

20. Knochenprothese (10) gemäß Anspruch 19, wobei der Teil des einen Abschnitts einen Pflock zum Einsetzen in eine entsprechende Bohrung, die mit dem Femur (14) assoziiert ist, beinhaltet und der Teil des anderen Abschnitts einen Pflock zum Einsetzen in eine entsprechende Bohrung, die mit der Tibia (818) assoziiert ist, beinhaltet.

21. Knochenprothese (10) gemäß Anspruch 19 oder 20, wobei ein Außenumfang des Teils des zweiten Abschnitts gemustert ist, um eine Weiterleitung von akustischen Wellen entlang einer Außenoberfläche des Teils zu fördern.

22. Knochenprothese (10) gemäß Anspruch 20 oder 21, wobei einer oder beide von dem ersten und dem zweiten Abschnitt einen weiteren Pflock zum Einsetzen in eine entsprechende Bohrung, die mit dem jeweiligen Knochensegment assoziiert ist, umfassen.

23. Knochenprothese (10) gemäß Anspruch 22, wobei der weitere Pflock mindestens einen Kanal zum Weiterleiten von akustischer Energie (30) dahindurch zum jeweiligen Knochensegment umfasst, um das Wachstum von Knochen in der entsprechenden Bohrung neben dem weiteren Pflock zu stimulieren und **dadurch** den weiteren Pflock in der entsprechenden Bohrung zu stabilisieren.

24. Knochenprothese (10) gemäß Anspruch 23, wobei der mindestens eine Kanal ein interner Kanal mit einer inneren reflektierenden Oberfläche (42) ist, die eine Resonanzkammer (34) definiert, welche durch den weiteren Pflock angeordnet ist.

25. Knochenprothese (10) gemäß Anspruch 24, wobei die Resonanzkammer (34) mindestens einen Durchlass zum Empfangen von akustischer Energie (30) umfasst.

26. Knochenprothese (10) gemäß einem der Ansprüche 23 bis 25, wobei auf dem weiteren Pflock eine Vielzahl von Oberflächenrillen bereitgestellt sind, um akustische Energie (30) durch die Rillen weiterzuleiten.

27. Knochenprothese (10) gemäß Anspruch 19, wobei der eine und der andere von dem ersten und dem zweiten Abschnitt Außenoberflächen, die drehend ineinander eingreifen, und dem Knochen zugewendete Innenoberflächen, die angepasst sind, um in den Femur (14) bzw. die Tibia (818) einzugreifen, umfassen.

28. Knochenprothese (10) gemäß Anspruch 27, wobei der eine Abschnitt im Allgemeinen U-förmig ist und angepasst ist, um die Patella des Femurs (14) zu umgreifen, und der andere Abschnitt im Allgemeinen T-förmig ist und angepasst ist, um oben auf die Tibia (818) zu passen.

**29.** Knochenprothese (10) gemäß Anspruch 27, wobei der eine und der andere von dem ersten und dem zweiten Abschnitt eine Vielzahl von Rillen umfassen, die sich entlang der dem Knochen zugewendeten Innenoberfläche davon befinden.

**30.** Knochenprothese (10) gemäß Anspruch 27, wobei die Außenoberfläche des anderen Abschnitts mindestens eine Aussparung zum Aufliegenlassen der Außenoberfläche des einen Abschnitts auf eine wiegenartige Weise umfasst.

**31.** Knochenprothese (10) gemäß Anspruch 1, in der das Muster ein senkrechtes Rillenmuster ist.

**32.** Knochenprothese (10) gemäß Anspruch 1, in der das Muster ein wabenförmiges Muster ist

**33.** Knochenprothese (10) gemäß Anspruch 1, in der das Muster ein halbkreisförmiges/Spiralmuster ist.


**Revendications**

**1.** Une prothèse osseuse (10) comportant une première portion (32) destinée à être insérée jusque dans un canal médullaire d'un premier segment d'os (14) ou un alésage dans celui-ci, ladite partie comprenant un canal interne, ledit canal interne ayant une surface réfléchissante intérieure (42) qui définit une chambre de résonance (34) disposée à travers ladite partie pour propager de l'énergie acoustique (30) au canal médullaire dudit premier segment d'os ou l'alésage dans celui-ci pour stimuler la croissance d'os dans ledit canal médullaire ou alésage adjacent à la partie et stabiliser de ce fait la partie dans ledit canal médullaire ou alésage, **caractérisée en ce que** la périphérie externe de la première portion est à motif pour favoriser la propagation d'onde acoustique le long de sa surface externe.

**2.** Une prothèse osseuse (10) selon la revendication 1, dans laquelle ladite chambre de résonance (34) comprend au moins une ouverture pour recevoir de l'énergie acoustique (30).

**3.** Une prothèse osseuse (10) selon la revendication 1 ou la revendication 2, dans laquelle ladite chambre de résonance (34) est convolutée.

**4.** Une prothèse osseuse (10) selon n'importe laquelle des revendications 1 à 3, laquelle comprend un transducteur (144) destiné à recevoir de l'énergie acoustique (32) et à émettre des ondes acoustiques (30) à travers ledit canal.

**5.** Une prothèse osseuse (10) selon la revendication 4, dans laquelle le transducteur (144) est un collier formant transducteur (546).

**6.** Une prothèse osseuse (10) selon la revendication 4, dans laquelle le transducteur (144) est disposé dans le canal.

**7.** Une prothèse osseuse (10) selon n'importe laquelle des revendications précédentes, dans laquelle un enduit poreux est fourni sur la partie.

**8.** Une prothèse osseuse (10) selon la revendication 7, dans laquelle un matériau formant membrane piézoélectrique est disposé entre ledit enduit poreux et une périphérie externe de ladite partie.

**9.** Une prothèse osseuse (10) selon la revendication 7, dans laquelle un matériau formant membrane piézocéramique est disposé entre ledit matériau poreux et une périphérie externe de ladite partie.

**10.** Une prothèse osseuse (10) selon n'importe laquelle des revendications précédentes, laquelle comprend une portion formant bille pour s'engager dans l'acétabulum de l'os pelvien (26) et ladite partie de la première portion est un implant destiné à s'engager dans le canal médullaire (38) du fémur (14).

**11.** Une prothèse osseuse (10) telle que revendiquée dans la revendication 1 dans laquelle le motif est un motif en zig-zag.

**12.** Une prothèse osseuse (10) selon la revendication 10, dans laquelle ladite chambre de résonance (34) comprend une pluralité de fentes qui s'étendent vers l'extérieur à partir de ladite chambre de résonance (34) pour transmettre de l'énergie acoustique (30) directement au canal médullaire (38).

13. Une prothèse osseuse (10) selon la revendication 1, comportant de plus une deuxième portion ayant une partie destinée à être insérée jusque dans un canal médullaire ou alésage dans un deuxième segment d'os.

14. Une prothèse osseuse (10) selon la revendication 13, dans laquelle ladite partie de la deuxième portion comprend au moins un canal destiné à propager de l'énergie acoustique à travers celle-ci au deuxième segment d'os pour stimuler la croissance d'os dans ledit canal médullaire (38) ou alésage adjacent à ladite partie et de ce fait stabiliser ladite partie dans le canal médullaire (38) du deuxième segment d'os ou de l'alésage dans celui-ci.

15. Une prothèse osseuse (10) selon la revendication 14 dans laquelle cedit au moins un canal est un canal interne ayant une surface réfléchissante intérieure (42) qui définit une chambre de résonance (34) disposée à travers ladite partie de la deuxième portion.

16. Une prothèse osseuse (10) selon la revendication 15 dans laquelle ladite chambre de résonance (34) comprend au moins une ouverture destinée à recevoir l'énergie acoustique.

17. Une prothèse osseuse (10) selon la revendication 13 dans laquelle une portion parmi la première portion et la deuxième portion est adaptée pour s'engager dans le canal médullaire (38) de l'humérus et l'autre portion parmi la première portion et la deuxième portion est adaptée pour s'engager dans le canal médullaire (38) de l'ulna, ladite première portion et ladite deuxième portion pouvant être déplacées l'une relativement à l'autre autour d'un pivot.

18. Une prothèse osseuse (10) selon la revendication 17 dans laquelle une périphérie externe de ladite partie de la deuxième portion est à motif pour favoriser la propagation d'onde acoustique le long d'une surface externe de ladite partie.

19. Une prothèse osseuse (10) selon la revendication 13, dans laquelle la portion parmi la première portion et la deuxième portion est adaptée pour s'engager dans le fémur (14) et l'autre portion parmi la première portion et la deuxième portion est adaptée pour s'engager dans le tibia (818), ladite première portion et ladite deuxième portion pouvant être déplacées l'une relativement à l'autre.

20. Une prothèse osseuse (10) selon la revendication 19, dans laquelle ladite partie de la portion comporte une cheville destinée à être insérée dans un alésage correspondant associé au fémur (14) et ladite partie de l'autre portion comporte une cheville destinée à être insérée dans un alésage correspondant associé au tibia (818).

21. Une prothèse osseuse (10) selon la revendication 19 ou la revendication 20, dans laquelle une périphérie externe de ladite partie de la deuxième portion est à motif pour favoriser la propagation d'onde acoustique le long d'une surface externe de ladite partie.

22. Une prothèse osseuse (10) selon la revendication 20 ou la revendication 21, dans laquelle soit la première portion, soit la deuxième portion ou les deux comprend une cheville supplémentaire destinée à être insérée dans un alésage correspondant associé au segment d'os respectif.

23. Une prothèse osseuse (10) selon la revendication 22, dans laquelle ladite cheville supplémentaire comprend au moins un canal destiné à propager de l'énergie acoustique (30) à travers celle-ci au segment d'os respectif pour stimuler la croissance d'os dans ledit alésage correspondant adjacent à ladite cheville supplémentaire et stabiliser de ce fait ladite cheville supplémentaire dans ledit alésage correspondant.

24. Une prothèse osseuse (10) selon la revendication 23, dans laquelle cedit au moins un canal est un canal interne ayant une surface réfléchissante intérieure (42) qui définit une chambre de résonance (34) disposée à travers ladite cheville supplémentaire.

25. Une prothèse osseuse (10) selon la revendication 24, dans laquelle ladite chambre de résonance (34) comprend au moins un orifice destiné à recevoir de l'énergie acoustique (30).

26. Une prothèse osseuse (10) selon n'importe laquelle des revendications 23 à 25, dans laquelle une pluralité de rainures de surface sont fournies sur la cheville supplémentaire pour propager de l'énergie acoustique (30) à travers les rainures.

27. Une prothèse osseuse (10) selon la revendication 19, dans laquelle ladite une portion et ladite autre portion parmi la première portion et la deuxième portion comprennent des surfaces externes qui s'engagent de façon pivotante les unes dans les autres et des surfaces internes faisant face à l'os qui sont adaptées pour s'engager dans le fémur (14) et le tibia (818), de façon respective.

28. Une prothèse osseuse (10) selon la revendication 27, dans laquelle ladite une portion est généralement configurée comme un U et est adaptée pour englober la rotule du fémur (14) et ladite autre portion est généralement configurée comme un T et est adaptée pour s'emboîter sur le dessus du tibia (818).

29. Une prothèse osseuse (10) selon la revendication 27 dans laquelle ladite portion et ladite autre portion parmi la première portion et la deuxième portion comprennent une pluralité de rainures situées le long de ladite surface interne faisant face à l'os de celle-ci.

30. Une prothèse osseuse (10) selon la revendication 27, dans laquelle ladite surface externe de ladite autre portion comprend au moins un renfoncement destiné à asseoir la surface externe de cette dite une portion de façon semblable à un berceau.

31. Une prothèse osseuse (10) telle que revendiquée dans la revendication 1 dans laquelle le motif est un motif à rainures verticales.

32. Une prothèse osseuse (10) telle que revendiquée dans la revendication 1 dans laquelle le motif est un motif en nid d'abeille.

33. Une prothèse osseuse (10) telle que revendiquée dans la revendication 1 dans laquelle le motif est un motif semi-circulaire/en spirale.

Fig. 1A

Fig. 1B

Fig. 1C

Fig. 2A

Fig. 2B

Fig. 3

Fig. 4

Fig. 5A

Fig. 5B

Fig. 6A

Fig. 6B

710

718

12

30

718

30

732

755

732

755

765

765

765

734

Fig. 7B

718

732

755

734 Fig. 7C

14

38

718

732

755

718

732

755

734 Fig. 7D

734 Fig. 7E

Fig. 7A

Fig. 8

Fig. 9

810a

30'

30'

814

820a

816

30

12

830a

FIG. 10A

30'

818

FIG. 10B

844a

847a

810a

844a

842a

848a

950a

850a

845a

840a

832a

B

B

A

A

820a

842a

848a

848a

842a

847a

FIG. 10C

840a

FIG. 10I

832a

830a

840a

845a

836a

FIG. 10D

FIG. 10J

FIG 10 E

FIG. 10F

FIG. 10G

FIG. 10H

FIG. 11A

FIG 11B

FIG. 12A

FIG. 12B

FIG. 12C

FIG. 13A

FIG. 13B

FIG. 13C